Europäisches Patentamt

European Patent Office

Office européen des brevets

(11) Publication number : **0 679 391 A2**

# EUROPEAN PATENT APPLICATION

(21) Application number : 95302924.6

(22) Date of filing : 28.04.95

(51) Int. Cl.⁶ : **A61K 9/30,** A61K 9/28, A61K 9/48

(30) Priority : **29.04.94 US 236591**

(43) Date of publication of application :
**02.11.95 Bulletin 95/44**

(84) Designated Contracting States :
**AT BE CH DE DK ES FR GB GR IE IT LI LU MC NL PT SE**

(71) Applicant : **McNEIL-PPC, INC.**
**Van Liew Avenue**
**Milltown New Jersey 08850 (US)**

(72) Inventor : **Sonley, Christopher R.**
**R.R. 6**
**Guelph, Ontario (CA)**
Inventor : **Turnbull, Mark Allen**
**1005-65 Speedvale Ave.**
**Guelph, Ontario (CA)**

(74) Representative : **Mercer, Christopher Paul et al**
**Carpmaels & Ransford**
**43, Bloomsbury Square**
**London WC1A 2RA (GB)**

(54) Solid pharmaceutical dosage form containing flavored film coating.

(57)    The present invention relates to a solid, swallowable pharmaceutical dosage form for temporary relief of coughs and a sore throat. The dosage form contains a solid core with a therapeutically effective amount of an analgesic and an antitussive. A menthol-flavored film coating is applied to the core in an amount sufficient to provide sensory stimulation of the throat and nasal passageways when the dosage form is placed in the oral cavity and swallowed.

EP 0 679 391 A2

Jouve, 18, rue Saint-Denis, 75001 PARIS

## FIELD OF THE INVENTION

The present invention relates to a solid pharmaceutical dosage form containing a pharmaceutical core coated with a menthol-flavored film.

## BACKGROUND OF THE INVENTION

Solid pharmaceutical dosage forms, such as tablets and caplets, have been coated for a variety of reasons. These coatings have been used to protect the active ingredient from the surrounding environment, e.g., air, moisture, or light, or to mask the unpleasant taste and/or odor of the pharmaceutical active. Thin coatings have also been used to improve the mechanical strength of the dosage form and to eliminate dusting during the manufacturing process, particularly during product packaging. The release properties of active ingredient can also be controlled through the use of a coating on the exposed surfaces of the tablet or caplet. Enteric and sustained-released coatings are examples of coatings that modify drug release.

One of the earliest forms of tablet coating is sugar coating. Sugar coating generally involves six steps, sealing, subcoating, smoothing, color coating, polishing and printing. Film coating, which involves the deposition of a thin polymeric film onto the dosage form from organic or water-based solutions, has become a popular alternative to sugar coating.

Aqueous dispersion containing a water soluble film forming polymer, a flavoring agent and a sweetening agent have been used to thinly coat a tablet containing an unpleasant tasting pharmaceutical. U.S. Patent No. 5,098,715 to McCabe et al. suggests that these types of coating can also be used to enable a patient to orally identify a tablet due to a particular flavor which is associated with the pharmaceutical found in the tablet core. This oral identification allows visually impaired as well as other persons to verify that the correct medication is being taken.

Certain flavoring agents are known to have a therapeutic value in pharmaceutical preparations. Menthol is used in cough and sore throat lozenges and cough syrups to provide temporary relief of minor throat irritation and coughs due to colds and inhaled irritants. Upon oral administration, the menthol, which is typically contained in a hard, candy-type base, provides a cooling sensation to irritated throat tissue and often makes the user's nasal passages feel clearer.

It would be desirable to formulate a swallowable, multi-symptom, cough/cold tablet which, in addition to containing an analgesic and an antitussive, also contains menthol for providing the temporary relief one normally associates with a cough and sore throat lozenge.

## SUMMARY OF THE INVENTION

The present invention provides a solid, swallowable pharmaceutical dosage form for temporary relief of coughs and sore throat. The dosage form contains a solid core with a therapeutically effective amount of an analgesic and an antitussive. A menthol-flavored film coating is applied to the core in an amount sufficient to provide sensory stimulation of the throat and nasal passageways when the dosage form is placed in the oral cavity and swallowed.

## DETAILED DESCRIPTION OF THE PREFERRED EMBODIMENTS

The solid pharmaceutical dosage form of the present invention provides temporary relief of a sore throat and coughing commonly associated with a cold or the flu. The dosage form should be of a size and shape so that it may be readily swallowed by a human. Preferably the dosage is in the shape of a tablet or caplet. The dosage form contains a solid core which is overcoated with a menthol-flavored thin film. When the dosage form is orally ingested, the menthol in the thin film coating provides sensory stimulation, such as a cooling sensation, of the throat and nasal passageway.

The core in the dosage form contains a therapeutically effective amount of an analgesic and an antitussive, and one or more pharmaceutically acceptable excipients. The analgesic is included in the dosage form for treating pain, e.g., sore throat, headache and muscle pain, that a patient suffering from a cold or the flu may be experiencing. The antitussive is used to inhibit or suppress the patient's coughing.

The analgesic used in the present invention includes acetaminophen and non-steroidal anti-inflammatory drugs (NSAIDs). Preferably, the NSAID is selected from the group consisting of propionic acid derivatives, such as ibuprofen, fenoprofen, naproxen and ketoprofen; fenamic acid derivatives, such as meclofenamate and mefenamic acid; oxicams, such as piroxicam; indole acetic acids, such as indomethacin, sulindac and tolmetin; asprin; and pharmaceutically acceptable salts thereof.

Suitable antitussives for use in the present invention include dextromethorphan, diphenhydramine, pharmaceutically acceptable salts thereof (e.g.,dextromethorphan hydrobromide and diphenhydramine hydrochloride), and mixtures thereof.

Additional pharmaceutical actives may be included in the core of the oral solid dosage form of the present invention. Suitable actives include expectorants, antihistamines, sympathomimetics and mixtures thereof. Expectorants which may be used in the invention include guaifenesin. Antihistamines suitable for use in the invention include chlorpheniramine, brompheniramine, terfenadine, astemizole, diphenhydramine, pharmaceutically acceptable salts thereof (e.g., chlorpheniramine maleate, brompheniramine maleate and diphenhydramine hydrochloride), and mixtures thereof. Sympathomimetics suitable for use in the concentrate include pseudoephedrine, phenylpropanolamine, pharmaceutically acceptable salts thereof (e.g., pseudoephedrine hydrochloride), and mixtures thereof.

Additional antitussives, expectorants, antihistamines, sympathomimetics, and analgesics suitable for use in the present invention are described in Remington's Pharmaceutical Sciences, Mack Publishing Co., Easton, PA, 18th ed., Chapters 39, 42, 43, 58 and 59 (1990), which is hereby incorporated by reference.

The pharmaceutical actives are present in the dosage form in a therapeutically effective amount, which is an amount that produces the desired therapeutic response upon oral administration, and can be readily determined by one skilled in the art. In determining such amounts, the particular compound being administered, the bio-availability characteristics of the compound, the dose regime, the age and weight of the patient, and other factors must be considered. In a preferred embodiment, the solid oral dosage form contains about 160 to about 500 mg acetaminophen, about 7.5 to about 15 mg dextromethorphan HBr, 0 to about 30 mg pseudoephedrine HCl and 0 to about 2 mg chlorpheniramine maleate.

The core is formed by mixing the pharmaceutical actives with one or more excipients, and then compressing the mixture into tablets or caplets using conventional tableting machines. The excipients used in the dosage form include those inert ingredients, e.g., diluents, binders, lubricants, glidants, disintegrants, etc., commonly found in solid oral dosage forms. Specific examples of pharmaceutically acceptable excipients that can be used to formulate the dosage form may be found in the Handbook of Pharmaceutical Excipients, published by the American Pharmaceutical Association and The Pharmaceutical Society of Great Britain, Washington, D.C. (1986); Pharmaceutical Dosage Forms: Tablets (edited by Lieberman et al.), 2nd. ed., Vols. 1-3, published by Marcel Dekker, Inc., New York (1989) and Remington's Pharmaceutical Sciences (edited by A. L. Gennaro), Mack Publishing Co., Easton, PA, 18th ed., Chapter 89 (1990), all of which are hereby incorporated by reference.

The menthol-flavored film coating is formed on at least a portion, preferably on all, of the exposed surface of the core containing the pharmaceutical actives. The film forming agent is typically a water soluble film forming polymer, such as hydroxpropyl methylcellulose, methylcellulose, ethylcellulose, hydroxypropyl cellulose, povidone, polydextrose, lactose, maltodextrin, acrylic polymer, and mixtures thereof. The film coating may optionally contain a plasticizer, such as polyethylene glycol, propylene glycol or glycerine, and a coloring or opacifying agent. A preferred blend of hydroxypropyl methylcellulose, a plasticizer and a colorant is commercially available from Colorcon, West Point, Pa. under the tradename OPADRY®. OPADRY Red is a red powder which is dispersed in water to form an aqueous film coating. This product contains hydroxypropyl methylcellulose, FD&C red no. 40 aluminum lake, polyethylene glycol, titanium dioxide, FD&C yellow no. 6 aluminum lake and polysorbate 80.

Suitable menthol flavoring agents for use in the film coating include natural and/or artificial mint and peppermint, eucalyptus oil, menthol crystals, mint oil, anise oil, and mixtures thereof. Peppermint NAEFCO, and natural and artificial mint flavors from Firmenich of Canada Ltd. Brampton, Ont., Canada are preferred. A sweetening agent is not employed as a flavoring agent since it is desirable to have the cooling sensation of menthol perceived by the patient, rather than the sweet flavor commonly found in candy mints.

The menthol-flavored film coating is applied to the cores as an aqueous dispersion and dried to form a thin film coating. The relative proportions of the ingredients in the coating are readily determined by one skilled in the art to achieve the desired sensory stimulation of the throat and nasal passageways. The dispersion will generally contain (w/w) about 5 to about 20 percent of the film forming polymer, about 2 to about 10 percent of the menthol flavoring agent, and about 70 to about 93 percent purified water.

The dispersion is applied to the cores using conventional pharmaceutical coating equipment, such as an Accela-Cota® coating pan from Thomas Engineering, Inc., Hoffman Estates, Ill. Other film coating techniques suitable for use in the present invention are described in Remington's Pharmaceutical Sciences (edited by A. L. Gennaro), Mack Publishing Co., Easton, PA, 18th ed., Chapter 90 (1990), which is hereby incorporated by reference.

The menthol-flavored film coating (dried) generally constitutes from about 1 to about 10, preferably about 2 to about 6, percent by weight of the total weight of the solid dosage form. A wax coating may also be applied over the menthol-flavored coating, but in a amount that will allow the patient to experience the cooling sensation of menthol when the dosage form is placed in the mouth and swallowed. This sensation is maintained for about 30 seconds to about 5 minutes, and preferably for at least about 2 minutes.

Specific embodiments of the present invention are illustrated by way of the following examples. This invention is not confined to the specific limitations set forth in these examples, but rather to the scope of the appended claims. Unless otherwise stated, the percentages and ratios given below are by weight.

**EXAMPLE I**

This Example discloses a regular strength caplet containing a menthol-flavored thin film coating. The ingredients contained in the caplet are as follows:

| Ingredient | Concentration (mg/dose) | Wt. Percent |
|---|---|---|
| Acetaminophen | 325.05 | 68.87 |
| Dextromethorphan HBr | 15.00 | 3.18 |
| Powdered Cellulose | 30.12 | 6.38 |
| Corn Starch (Pregel) | 14.44 | 3.06 |
| Sodium Starch Glycolate | 13.10 | 2.78 |
| Corn Starch (Modified) | 26.67 | 5.65 |
| Magnesium Stearate | 2.13 | 0.45 |
| Microcrystalline Cellulose | 20.00 | 4.24 |
| Hydroxpropyl methyl-cellulose (OPADRY Red YS-1-1833) | 17.86 | 3.78 |
| Carnauba Wax | 0.01 | 0.003 |
| Peppermint NAEFCO | 4.47 | 0.95 |
| Natural and Artificial Mint Flavors | 3.13 | 0.66 |

The caplet cores were prepared as follows:

1. A granulation was prepared by spraying modified starch onto a fluidizied bed of acetaminophen, dextromethorphan HBr, powdered cellulose, pregelatinized starch, and sodium starch glycolate.
2. The above granulation was then dry blended with microcrystalline cellulose and magnesium stearate.
3. The mixture was compressed using a Manesty BB4 rotary tablet press into caplet-shaped tablets.
4. A coating solution having the following composition (w/w) was prepared:

| | |
|---|---|
| OPADRY Red | 9.6% |
| Peppermint NAEFCO | 2.4% |
| Natural and Artificial Mint Flavor | 1.7% |
| Purified Water | 86.3% |

5. Ten (10) kg of the caplet cores were placed in a 24 inch Accela-Cota coating pan rotating at 12 rpm. The coating dispersion was applied using an air atomized sprayer and standard coating procedures. Once the coating was complete, the caplet bed was waxed by sprinkling with powdered carnauba wax and rotating until uniform. The coated caplets exhibited a sealed and polished appearance.

Caplets with this coating exhibited a warming menthol-like smell and flavor which stimulates the nasal passageways and throat when the caplet was placed in the mouth and swallowed.

**EXAMPLE II**

This Example discloses a extra strength caplet containing a menthol-flavored thin film coating. The ingredients contained in the caplet are as follows:

| Ingredient | Concentration (mg/dose) | Wt. Percent |
|---|---|---|
| Acetaminophen (directly compressible) | 551.42 | 82.31 |
| Dextromethorphan HBr | 15.01 | 2.24 |
| Sodium Starch Glycolate | 15.75 | 2.35 |
| Magnesium Stearate | 3.17 | 0.47 |
| Microcrystalline Cellulose | 47.48 | 7.09 |
| Hydroxpropyl methyl-cellulose (OPADRY Red YS-1-1833) | 26.26 | 3.92 |
| Carnauba Wax | 0.02 | 0.003 |
| Peppermint NAEFCO | 6.35 | 0.95 |
| Natural and Artificial Mint Flavors | 4.44 | 0.66 |

The caplet cores were prepared as follows:
1. The directly compressible acetaminophen was dry blended in stages with microcrystalline cellulose, dextromethorphan HBr, sodium starch glycolate, and magnesium stearate.
2. The blend was then compressed using a Manesty BB4 rotary tablet press into caplet-shaped tablets.
3. A coating solution having the following composition (w/w) was prepared:

| OPADRY Red | 9.6% |
|---|---|
| Peppermint NAEFCO | 2.4% |
| Natural and Artificial Mint Flavor | 1.7% |
| Purified Water | 86.3% |

4. Ten (10) kg of the caplet cores were placed in a 24 inch Accela-Cota coating pan rotating at 12 rpm. The coating dispersion was applied using an air atomized sprayer and standard coating procedures. Once the coating was complete, the caplet bed was waxed by sprinkling with powdered carnauba wax and rotating until uniform.
The coated caplets exhibited a sealed and polished appearance.

Caplets with this coating exhibited a warming menthol-like smell and flavor which stimulates the nasal passageways and throat when the caplet was placed in the mouth and swallowed.

**EXAMPLE III**

This Example discloses a placebo caplet containing a menthol-flavored thin film coating. The ingredients contained in the caplet are as follows:

| Ingredient | Concentration (mg/Dose) | Wt.% |
|---|---|---|
| White Compressible Sugar | 564.06 | 76.53 |
| Microcrystalline Cellulose | 142.80 | 19.38 |
| Magnesium Stearate | 7.14 | 0.97 |
| OPADRY Red | 16.13 | 2.19 |
| Natural and Artificial Mint Flavor | 2.82 | 0.38 |
| Peppermint NAEFCO | 4.03 | 0.55 |
| Carnuba Wax | 0.01 | 0.001 |

The placebo caplet cores were prepared as follows:

1. A granulation was prepared by dry mixing the white compressible sugar, microcrystalline cellulose and magnesium stearate.

2. The above mixture was then compressed using a Manesty BB4 rotary tablet press.

3. A coating solution having the following composition (w/w) was prepared:

| OPADRY Red | 9.6% |
|---|---|
| Peppermint NAEFCO | 2.4% |
| Natural and Artificial Mint Flavor | 1.7% |
| Purified Water | 86.3% |

4. 9.4 kg of the placebo caplet cores were placed in a 24 inch Accela-Cota coating pan rotating at 12 rpm. The coating dispersion was applied using an air atomized sprayer and standard coating procedures. Once the coating was complete the caplet bed was waxed by sprinkling with powdered carnuba wax and rotating until uniform. The coated caplets exhibited a sealed and polished appearance. The menthol-flavored coating constituted 3.2 percent by weight of the total weight of the placebo caplet.

Caplets with this coating exhibited a warming menthol-like smell and flavor which stimulated the nasal passageways and throat when the caplet was placed in the mouth and swallowed. A 20 person taste trial was conducted where each participant swallowed one of the placebo caplets containing the menthol-flavored coating. The results of the taste test are as follows:

| Duration of Stimulation | Less than 1 Minute | 1-2 Minutes | Greater than 2 Minutes |
|---|---|---|---|
| Frequency | 3 | 8 | 9 |

These results show that stimulation was maintained for an average of 1-2 minutes in the taste trial.

## EXAMPLE IV

This Example discloses a regular strength caplet as in EXAMPLE I with the addition of pseudoephedrine. A coating dispersion of the following percentages (w/w) is prepared:

| OPADRY Yellow | 11.1% |
|---|---|
| Peppermint NAEFCO | 2.4% |
| Natural and Artificial Mint Flavors | 1.7% |
| Purified Water | 84.8% |

Ten (10) kg of caplet cores, each containing the active ingredients:
Acetaminophen (325 mg)
Pseudoephedrine HCl (30 mg)
Dextromethorphan HBr (15 mg)
and suitable excipients are prepared using procedures similar to those described in EXAMPLE I. The caplet cores are placed in a 24 inch Accela-Cota coating rotating at 12 rpm. The coating dispersion is applied using an air atomized sprayer and standard coating procedures. The caplets are then sealed and polished with carnauba wax.

## EXAMPLE V

This Example discloses a regular strength caplet as in EXAMPLE I with the addition of pseudoephedrine and chlorpheniramine maleate. A coating dispersion of the following percentages (w/w) is prepared:

| OPADRY Yellow | 11.1% |
|---|---|
| Peppermint NAEFCO | 2.4% |
| Natural and Artificial Mint Flavors | 1.7% |
| Purified Water | 84.8% |

Ten (10) kg of caplet cores, each containing the active ingredients:
Acetaminophen (325 mg)
Pseudoephedrine HCl (30 mg)
Dextromethorphan HBr (15 mg)
Chlorpheniramine maleate (2 mg)
and suitable excipients are prepared using procedures similar to those described in EXAMPLE I. The caplet cores are placed in a 24 inch Accela-Cota coating rotating at 12 rpm. The coating dispersion is applied using an air atomized sprayer and standard coating procedures. The caplets are then sealed and polished with carnauba wax.

Various modifications can be made to the above-described embodiments without departing from the spirit and scope of the present invention.

## Claims

1. A solid, swallowable pharmaceutical dosage form for temporary relief of coughs and sore throat, comprising:

   a solid core comprising a therapeutically effective amount of an analgesic and an antitussive; and
   a menthol-flavoured film coating disposed on at least a portion of said core, said coating comprising a water soluble, film forming agent and a menthol flavouring agent in an amount sufficient to provide sensory stimulation of the throat and nasal passageways when said dosage form is placed in the oral cavity and swallowed.

2. The dosage form of Claim 1, wherein said analgesic is acetaminophen or a non-steroidal anti-inflammatory drug.

3. The dosage form of Claim 2, wherein said analgesic is acetaminophen.

4. The dosage form of any one of Claims 1 to 3 wherein the antitussive is dextromethorphan, diphenhydra-

7

mine, a pharmaceutically acceptable salt thereof or a mixture thereof.

5. The dosage form of Claim 4, wherein the antitussive is dextromethorphan HBr.

6. The dosage form of any one of Claims 1 to 5, wherein said solid core contains at least one pharmaceutically acceptable excipient.

7. The dosage form of any one of Claims 1 to 6, wherein said film forming agent is a polymer.

8. The dosage form of Claim 7 wherein said film forming agent is a water soluble film forming polymer which is hydroxpropyl methylcellulose, methylcelluose, ethylcellulose, hydroxypropyl cellulose, povidine, polydextrose, lactose, maltodextrin, acrylic polymer, or a mixture thereof.

9. The dosage form of Claim 8, wherein said film forming polymer comprises hydroxypropyl methylcellulose and a plasticizer.

10. The dosage form of any one of Claims 1 to 9, wherein said coating comprises 1 to 10 percent by weight of the total weight of the dosage form.

11. The dosage form of any one of Claims 1 to 10 wherein said menthol flavoring agent is a natural or artificial mint flavorant, a natural or artificial peppermint flavorant, menthol crystals, anise oil, eucalyptus oil, or a mixture thereof.

12. The dosage form of any one of Claims 1 to 11 wherein said film coating is free of any sweetening agent.

13. The dosage form of any one of Claims 1 to 12, wherein said stimulation is maintained for at least 30 seconds.